Europäisches Patentamt

European Patent Office

Office européen des brevets

(11) Publication number: **0 211 626**
**B1**

# EUROPEAN PATENT SPECIFICATION

(45) Date of publication of patent specification: **25.07.90**

(51) Int. Cl.⁵: **C 12 N 11/00, C 12 M 1/40**

(21) Application number: **86305920.0**

(22) Date of filing: **01.08.86**

(54) **Process of preparing immobilized enzymes and apparatus for performing the process.**

(30) Priority: **06.08.85 JP 171786/85**

(43) Date of publication of application:
**25.02.87 Bulletin 87/09**

(45) Publication of the grant of the patent:
**25.07.90 Bulletin 90/30**

(84) Designated Contracting States:
**CH DE FR GB IT LI NL**

(56) References cited:
**DE-A-2 929 872    GB-A- 794 855**
**DE-A-3 012 233    GB-A-2 098 236**
**DE-A-3 339 764**

**FOUNDATION OF BIOCHEMICAL
ENGINEERING; KINETICS AND
THERMODYNAMICS IN BIOLOGICAL
SYSTEMS; ACS SYMPOSIUM SERIES 207;
AMERICAN CHEMICAL SOCIETY, Washington,
D.C. 1983 J.KLEIN et al. "Immobilized cells;
Catalyst preparation and reaction performance"
pages 377-392**

(73) Proprietor: **MITSUI TOATSU CHEMICALS, Inc.
2-5 Kasumigaseki 3-chome
Chiyoda-Ku Tokyo 100 (JP)**

(72) Inventor: **Iguchi, Seiya
4-21-7, Kamirenzyaku
Mitaka-shi Tokyo (JP)**
Inventor: **Noguchi, Takeshi
2070, Lijimachou Totsuka-ku
Yokohama-shi Kanagawa-ken (JP)**
Inventor: **Kimura, Hiroshi
Haseryo, Mitsui Toatsu 4-1-28, Hase
Kamakura-shi Kanagawa-ken (JP)**
Inventor: **Aihara, Masayo
1-9-1, Horinouchichou Minami-ku
Yokohama-shi Kanagawa-ken (JP)**

(74) Representative: **Harvey, David Gareth et al
Graham Watt & Co. Riverhead
Sevenoaks Kent TN13 2BN (GB)**

## Description

This invention relates to a process for preparing immobilized enzymes in a short period of time and in large amounts, and to an apparatus useful for carrying out the process. This invention is applied e.g. to the preparation of immobilized enzymes used in the operation of a bioreactor.

Techniques for preparing immobilized enzymes to be used in a bioreactor are known, for example, a method wherein liquid drops or droplets are produced by forcing an enzyme-containing liquid from the tip of a small nozzle under a given pressure using a compressed gas and the entrapping immobilization method using carrageenan as disclosed by Chibata et al, Biotechnology and Bioengineering, Vol. XXI, pp. 1697—1709 (1979) John Wiley & Sons Inc; see also U.S. 4,138,292.

In these methods, a mixture of a sodium alginate solution or an aqueous carrageenan solution with a microorganism is dropped into an aqueous solution of a gelling agent such as calcium chloride to produce entrapped microorganism gel granules. These methods are however disadvantageous in the granulation ability in the case where the gel granules are to be produced in large quantities. They also involve a problem in that suspended drops are formed on the jet of the nozzle, requiring occasional wiping of the tip of the nozzle. Thus, neither of these methods has been regarded as appropriate for treating large amounts of microorganism in a short period of time.

It is an object of the present invention to provide a process and apparatus for preparing a large quantity of an immobilized enzyme or microorganism in a short period of time. The present invention provides a novel process and apparatus for rapidly preparing gel granules of an immobilized microorganism or immobilized enzyme in large amounts. Because a large amount of the immobilized gel granules can be produced very rapidly, the short period of time involved in the immobilization operation makes it feasible to minimize denaturing or the deactivation of enzymatic activity during the operation.

According to the present invention, there is provided a process for preparing an immobilized enzyme or microorganism with a carrier which comprises forming liquid drops or droplets of an enzyme or microorganism containing liquid from the periphery of a rotating disc by centrifugal force and bringing them into contact with a solidifying solution.

Also according to the present invention, there is provided an apparatus for preparing an immobilized enzyme or microorganism, which comprises a vessel for containing an enzyme or microorganism containing liquid and provided with a liquid feed pipe for transferring the liquid, a disc capable of rotating over which disc the outlet of the liquid feed pipe opens, and a vessel for containing a solution capable of solidifying liquid drops or droplets which in use fly from the periphery of the disc, to receive the liquid drops or droplets at the surface of the solution.

In practising a process according to the present invention, a crude enzyme extract or a microorganism is mixed with a carrier solution, the liquid mixture e.g. the enzyme-containing liquid, is fed to a rotating disc for ejection as liquid drops or droplets from the periphery of the disc, and the liquid drops or droplets are brought into contact with an aqueous solution of a gelling agent or a solidifying solution, thereby preparing an immobilized enzyme or microorganism. An apparatus for performing the process, can be provided with a vessel for preparing, storing and supplying the microorganism or enzyme-containing liquid, a rotating disc for releasing the liquid by centrifugal force from its periphery as liquid drops or droplets, and a vessel for bringing the released liquid drops or droplets into contact with a gelling solution. The rotating disc used in this apparatus is preferably characterised by saw-toothed cuts in its periphery, and advantageously a plurality of such rotating discs are provided in this apparatus.

The invention will now be explained in more detail in the following description, which is given by way of example, of preferred embodiments; the description is to be read in conjunction with the accompanying drawings, in which:

Figure 1 illustrates apparatus for effecting the process according to the present invention,

Figure 2 illustrates a disc having saw-toothed cuts in its periphery, and

Figure 3 illustrates an apparatus which uses a plurality of discs and is of the wetted-wall type.

In each of the Figures reference numbers 1, 2, 3, 4 and 5 represent an enzyme-containing liquid, a liquid feed pipe, a disc, a solidifying solution and a wetted wall column, respectively.

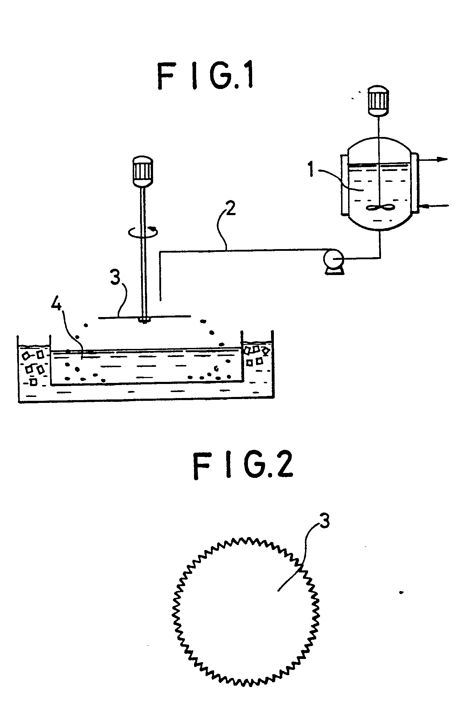

In Figure 1, a disc 3 fixed to the rotating axis of a motor is provided over a sufficiently large vessel containing a gelling solution 4. The vessel containing the gelling solution is cooled in a cold water bath generally kept cold with ice. Separately, an enzyme-containing liquid, or a mixed liquid of an enzyme or a microorganism holding an enzyme with a carrier solution, is stirred and prepared in a liquid feed tank. It is preferable to cool the liquid feed tank for example by means of a cooling jacket in order to maintain the enzymatic activity of the feed liquid. The enzyme-containing liquid is allowed to flow down onto a rotating disc, preferably in the vicinity of the centre thereof. The liquid is delivered via a liquid feed pipe 2, e.g. by means of a pump. Then, the enzyme-containing liquid on the rotating disc is caused to flow in radial directions to the disc periphery by centrifugal force. The liquid finally flies from the periphery and falls into the gelling solution in which gel granules are formed by a gellation reaction.

Figure 2 illustrates a rotating disc which has saw-toothed cuts around its periphery. A serrated disc of this form can be used in practising the present invention.

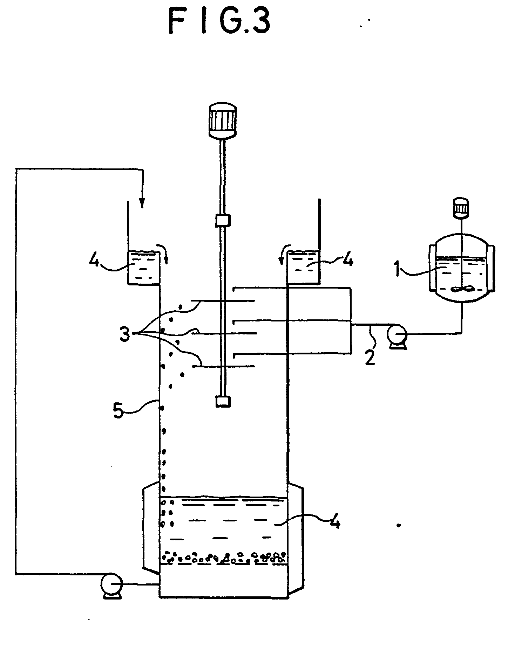

Figures 1 and 2 show the fundamental principle of the process and apparatus of the present invention, while Figure 3 illustrates an exemplary apparatus for the mass production of immobilized granules in accordance with the present invention. In the apparatus of Figure 3, a plurality of rotating discs are used. Thus, a plurality of rotating discs are fixed to the motor-driven rotating shaft, and each disc has an associated liquid feed unit for feeding the enzyme-containing liquid to that disc.

The gelling solution may be held in a vessel set under the disc as shown in Figure 1. Alternatively, the gelling solution may be allowed or caused to flow down as a curtain, e.g. as a wetted wall 5, around the disc, see for instance the wetted wall column shown in Figure 3.

In Figure 3, the gelling solution caused to flow down to the bottom of the column is separated from the granules formed thereby and is circulated back to the top of the wetted wall column so that it may be repeatedly used as a gelling solution. Some fresh gelling solution may also be added. This scheme is particularly effective in producing an immobilized microorganism gel in large amounts.

Microorganisms which may be used include common yeasts such as *Candida* and *Saccharomyces* and common bacteria such as *Pseudomonas, Escherichia coli, Norcadia, Gluconobacter* and *Zymomonas.* No particular limitations are imposed on the species. Also, no particular restrictions are placed on the variety of enzymes.

As the carrier for an enzyme or microorganism to be immobilized, an alginate solution and carrageenan, among others, are well known and widely applied. It is a matter of course that no limitations are vested on their kind in the present invention.

When practising the present invention, each immobilized microorganism or enzyme granule has an average size of approximately 0.5—6 mm. Proper selection of the diameter and revolution of the rotating disc makes it possible to produce granules of desired sizes. Generally, granules of 2—3 mm in size are widely used.

An aqueous solution of calcium chloride is generally used as the gelling solution.

The immobilized enzyme granule is not always a perfect sphere but frequently has the form of a drop or droplet. The aspect ratio of the granules is in the order of 1.6 to 3.0, which causes no particular inconvenience in practical use.

It is generally preferable that the gel formed from the liquid falling into the gelling solution after flying from the rotating disc be aged by being brought into contact with the gelling solution for a certain period of time so that the gellation is further advanced.

The present invention is further described by way of the following Examples. It should however be understood that the present invention is not limited to or by these Examples.

Example 1

The following is the case wherein an immobilized microorganism is prepared using the apparatus shown in Figure 1 and the rotating disc illustrated in Figure 2.

*Escherichia coli* was cultivated in a culture medium containing glucose and collected by using a centrifuge. The thus-collected wet microorganism was mixed with an equal amount by weight of an isotonic sodium chloride solution and further with four times as much of a 6 wt.% aqueous sodium alginate solution, thereby preparing a suspension of the microorganism.

The resulting suspension was charged in a vessel and kept at about 20°C. Separately, a 0.5 mol per liter aqueous calcium chloride solution was used as a gelling solution.

The suspension was fed onto a rotating disc by a pump and then allowed to fall into the gelling solution cooled at about 10°C as drops or droplets, and they were then aged in the gelling solution for about one hour. After the aging, the granules were taken out of the gelling solution and their granular sizes were measured. The results are shown in Table 1. The diameter of the disc used in the experiment was 150 mm, the number of teeth was 70, and the tooth depth was 9 mm.

### Table 1

### Results of Preparation Test of Immobilized

### Microorganism Using Rotating Disc

| Liquid Feed Rate | Disc Revolution | Average Granular Size | Aspect Ratio of Granules |
|---|---|---|---|
| g/H | rpm | mm | - |
| 250 | 155 | 3.9 | 1.8 |
|  | 177 | 3.4 | 2.0 |
|  | 236 | 3.3 | 2.5 |
|  | 311 | 2.5 | 2.3 |
|  | 430 | 1.6 | 1.7 |
| 1,883 | 150 | 6.4 | 2.4 |
|  | 300 | 4.8 | 2.8 |
|  | 390 | 2.4 | 1.7 |
|  | 450 | 2.0 | 2.2 |
| 3,320 | 250 | 4.9 | 2.8 |
|  | 320 | 4.1 | 2.7 |
|  | 390 | 3.7 | 3.1 |
|  | 433 | 2.5 | 2.3 |

### Example 2

An immobilized microorganism was prepared using the apparatus employed in Example 1 except that a disc with no saw-toothed cuts in its periphery was used. When the liquid feed rate was low, results similar to those of Example 1 were obtained.

### Example 3

The apparatus illustrated in Figure 3 was equipped with three discs similar to that used in Example 1 to prepare an immobilized microorganism.

The experiment was conducted at a liquid feed rate of 10 kg/H and a disc revolution of 420 rpm, using a 0.5 mol per liter aqueous calcium chloride solution as a gelling solution. After 30 minutes of operation, an immobilized microorganism having an average granular size of 2.6 mm was obtained in an amount of 4.2 kg.

**Claims**

1. A process for preparing an immobilized enzyme or microorganism with a carrier which comprises forming liquid drops or droplets of an enzyme or microorganism containing liquid from the periphery of a rotating disc by centrifugal force and bringing them into contact with a solidifying solution.

2. A method according to claim 1, wherein the drops or droplets are produced at the serrated edge of a disc having a toothed periphery.

3. A method according to claim 1 or claim 2, wherein the drops or droplets are flung outwardly by the disc into the solidifying solution while the latter flows past the disc as an encircling curtain, said solution being continuously recycled.

4. An apparatus for preparing an immobilized enzyme or microorganism, which comprises a vessel for containing an enzyme or microorganism containing liquid and provided with a liquid feed pipe for transferring the liquid, a disc capable of rotating over which disc the outlet of the liquid feed pipe opens, and a vessel for containing a solution capable of solidifying liquid drops or droplets which in use fly from the periphery of the disc, to receive the liquid drops or droplets at the surface of the solution.

5. An apparatus according to claim 4, wherein the disc has saw-toothed cuts in the periphery thereof.

6. An apparatus according to claim 4 or claim 5, wherein a plurality of discs are fixed to a common rotating axis.

7. An apparatus according to any of claims 4 to 6, wherein the vessel which receives the liquid drops or droplets has a wall surrounding the periphery of the disc and supply means is provided for the solidifying solution in such a manner that the wall constitutes a wetted wall of the solidifying solution.

## Patentansprüche

1. Verfahren zur Herstellung eines immobilisierten Enzyms oder Mikroorganismus mit einem Träger, das darin besteht, daß Flüssigkeitstropfen oder -tröpfchen einer ein Enzym oder einen Mikroorganismus enthaltenden Flüssigkeit vom Umfang einer rotierenden Scheibe aus durch Zentrifugalkraft gebildet und diese mit einer Verfestigungslösung in Kontakt gebracht werden.

2. Verfahren nach Anspruch 1, bei dem die Tropfen oder Tröpfchen an dem gezackten Rand einer einen gezahnten Umfang aufweisenden Scheibe erzeugt werden.

3. Verfahren nach Anspruch 1 oder Anspruch 2, bei dem die Tropfen oder Tröpfchen von der Scheibe nach außen in die Verfestigungslösung geschleudert werden, während letztere an der Scheibe als ein Ummantelungsvorhang vorbeiströmt, wobei die Lösung ständig im Umlauf geführt wird.

4. Vorrichtung zur Herstellung eines immobilisierten Enzyms oder Mikroorganismus, bestehend aus einem Gefäß für die Aufnahme einer ein Enzym oder einen Mikroorganismus enthaltenden Flüssigkeit, das mit einer Flüssigkeitszuführleitung für eine Übertragung der Flüssigkeit versehen ist, einer drehbaren Scheibe, über der der Auslaß der Flüssigkeitszuführleitung ausmündet, und einem Gefäß zur Aufnahme einer Lösung, die zur Verfestigung von Flüssigkeitstropfen oder -tröpfchen in der Lage ist, die im Betrieb von Umfang der Scheibe abfliegen, um die Flüssigkeitstropfen oder -tröpfen an der Oberfläche der Lösung aufzufangen.

5. Vorrichtung nach Anspruch 4, bei dem die Schiebe sägezahnförmige Einschnitte an ihrem Umfang aufweist.

6. Vorrichtung nach Anspruch 4 oder Anspruch 5, bei der mehrere Scheiben auf einer gemeinsamen rotierenden Achse festgelegt sind.

7. Vorrichtung nach einem Ansprüche 4 bis 6, bei dem das Gefäß, das die Flüssigkeitstropfen oder -tröpfchen aufnimmt, eine den Umfang der Scheibe umgebende Wand aufweist und eine Zuführeinrichtung für die Verfestigungslösung vorgesehen ist, derart, daß die Wand eine mit der Verfestigungslösung benetzte Wand bildet.

## Revendications

1. Procédé de préparation d'une enzyme ou d'un micro-organisme immobilisé avec un support, comprenant les opérations consistant à former des gouttes ou gouttelettes liquides d'une enzyme ou d'un micro-organisme sous l'effet de la force centrifuge à partir de la périphérie d'un disque en rotation et à les mettre en contact avec une solution solidifiante.

2. Procédé selon la revendication 1, dans lequel les gouttes ou gouttelettes sont produites au niveau du bord crénelé d'un disque ayant une périphérie dentée.

3. Procédé selon la revendication 1 ou 2, dans lequel les gouttes ou gouttelettes sont projetées vers l'extérieur par le disque dans la solution solidifiante, tandis que cette dernière s'écoule en regard du disque sous la forme d'un rideau qui l'encercle, cette solution étant recyclée de façon continue.

4. Appareil pour la préparation d'une enzyme ou d'un micro-organisme immobilisé, comprenant un récipient destiné à contenir un liquide (1) contenant l'enzyme ou le micro-organisme et muni d'un tuyau d'alimentation en liquid (2) pour le transfert du liquide, un disque (3) qui est susceptible de tourner et au-dessus duquel s'ouvre la sortie du tuyau d'alimentation en liquide (2), et un récipient destiné à contenir une solution (4) capable de solidifier les gouttes ou gouttelettes du liquide qui, en service, volent à partir de la périphérie du disque (3), afin de recevoir les gouttes ou gouttelettes du liquide à la surface de la solution (4).

5. Appareil selon la revendication 4, dans lequel le disque (3) comporte des entailles en dents de scie à sa périphérie.

6. Appareil selon la revendication 4 ou 5, dans lequel plusieurs disques (3) sont fixés à un axe de rotation commun.

7. Appareil selon l'une quelconque des revendications 4 à 5, dans lequel le récipient qui reçoit les gouttes ou gouttelettes présente une paroi (5) qui entoure la périphérie du disque (3), et en ce que des moyens d'alimentation sont prévus pour la solution solidifiante (4) de telle manière que cette paroi constitue une paroi à ruissellement de la solution solidifiante.

# F I G.1

# F I G.2

# FIG.3